Europäisches Patentamt

European Patent Office

Office européen des brevets

(11) Publication number: **0 340 176**
**A2**

## EUROPEAN PATENT APPLICATION

(21) Application number: **89830103.1**

(22) Date of filing: **07.03.89**

(51) Int. Cl.⁴: **A 61 F 2/46**

(30) Priority: **29.04.88 IT 2040288**

(43) Date of publication of application:
**02.11.89 Bulletin 89/44**

(84) Designated Contracting States:
**AT BE CH DE ES FR GB GR LI NL SE**

(71) Applicant: **G. CREMASCOLI S.p.A.**
**Via Clemente Prudenzio, 14/16**
**I-20138 Milano (IT)**

(72) Inventor: **Ghisellini, Franco G. Cremascoli S.p.A.**
**Via Clemente Prudenzio, 14/16**
**I-20138-Milano (IT)**

(74) Representative: **Cicogna, Franco**
**Ufficio Internazionale Brevetti Dott.Prof. Franco Cicogna**
**Via Visconti di Modrone, 14/A**
**I-20122 Milano (IT)**

(54) Device for the resection of a femoral bone for the application of knee articulation prostheses.

(57)

## DEVICE FOR THE RESECTION OF A FEMORAL BONE FOR THE APPLICATION OF KNEE ARTICULATION PROSTHESES

There is disclosed a device for the resection of the front,rear and transverse portions of the head of a femur in order to apply to the knee an articulation prosthesis.

This device essentially comprises a first parallelepipedal body adapted to be affixed to the distal part of the femur in order to resect the femur bone at the front and rear thereof,and a second body, cooperating with the first so as to transversely resect the distal part.

Fig. 2

**Description**

## BACKGROUND OF THE INVENTION

The present invention relates to a device for the resection of a femoral bone for the application of a knee articulation prosthesis.

As is known, frequently the knee articulation must be replaced by an artificial prosthesis, because, for example, of arthritis, tumoral deseases and the like.

In this case, the head portion of the femur must be resected in order to properly fit thereon a femoral prosthesis.

## SUMMARY OF THE INVENTION

The task of the present invention is to provide a device for the resection of a femoral bone which is specifically designed and arranged to apply several types of different femoral prosthesis.

Within the scope of this task, a main object of the present invention is to provide such a femoral resection device which can be easily fitted to the inclination of the diaphysis-femur axis of the patient, with respect to a vertical axis.

Another object of the present invention is to provide such a femoral resection device which is construction-wise very simple, can be simply operated and is very reliable in oparation.

According to one aspect of the present invention, the above mentioned task and objects, as well as yet other objects, which will become more apparent hereinafter, are achieved by a femoral resection device characterized in that said device essentially comprises a first shaped body, having a substantially parallelepipedal shape, adapted to be affixed on the distal part of a femur for the resection of the front and rear portions thereof, and a second shaped body, cooperating with the first shaped body and coupled thereto for transversely resecting said distal part of said femur.

## BRIEF DESCRIPTION OF THE DRAWINGS

Further characteristics and advantages of the device according to the present invention, will become more apparent from the following detailed description of a preferred embodiment thereof, which is illustrated, by way of an indicative but not limitative example, in the figures of the accompanying drawings, in which:

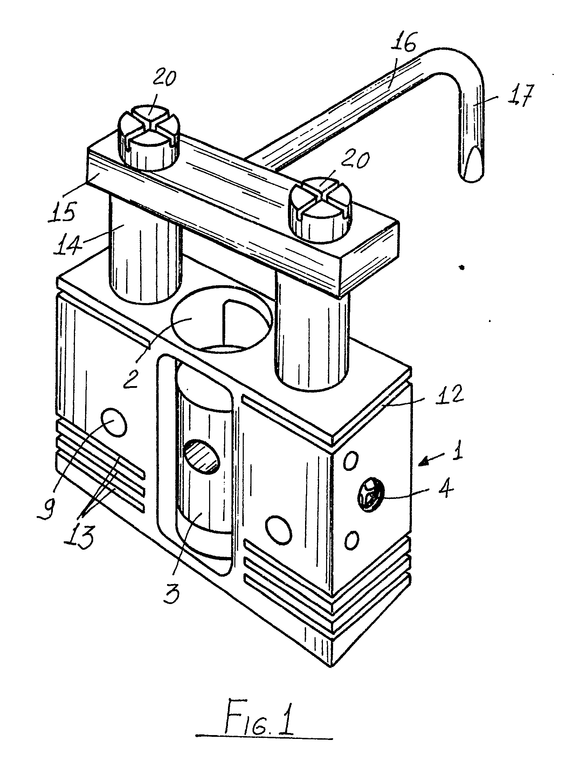

figure 1 shows a first shaped body included in the device according to the invention, said body including a lug which can operate as a reference member and contact the portion of a femur bone upstream of the distal part thereof;

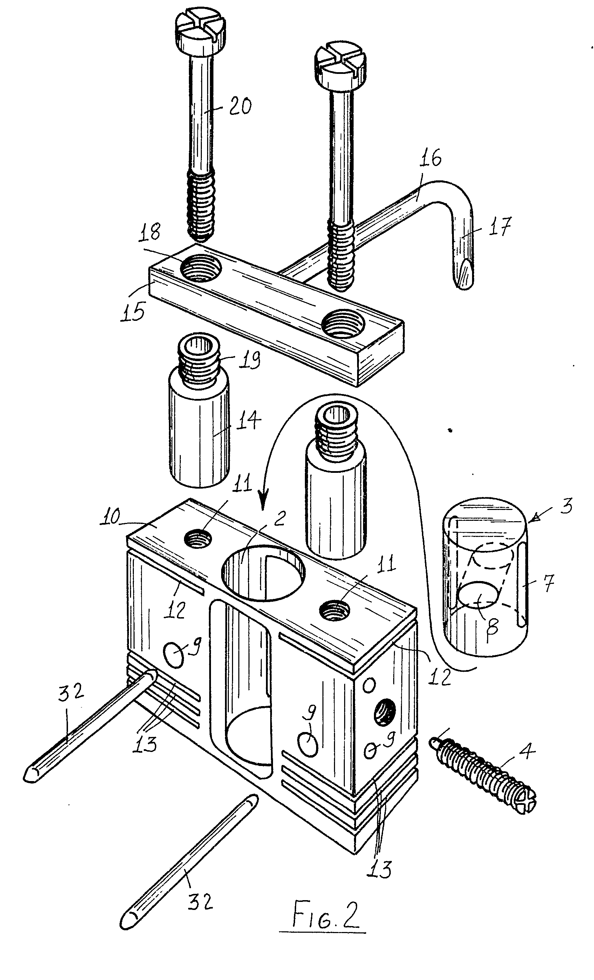

figure 2 is an exploded view illustrating the first shaped body;

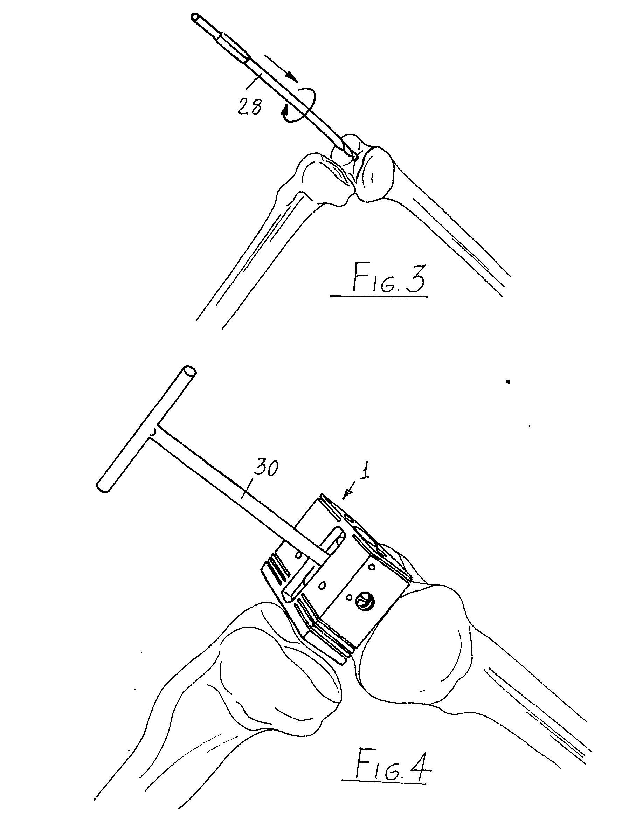

figures 3 to 7 illustrate a possible procedure for applying the above first shaped body on the front portion of the femur for resecting the front and rear portion thereof;

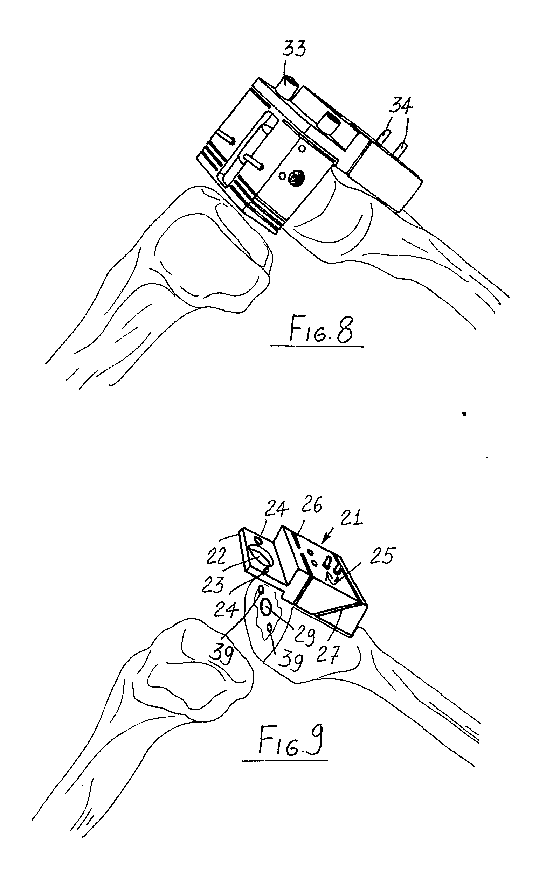

figures 8 and 9 schematically illustrate a possible procedure for using a second shaped body included in the resection device according to the invention;
and

figures 10 and 11 illustrate a femoral prosthesis respectively in its disengaged condition and in its condition engaged on the head of the femoral bone .

## DESCRIPTION OF THE PREFERRED EMBODIMENT

With reference to the figures of the accompanying drawings, the femoral resection device according to the present invention comprises a first shaped body 1, of parallelepipedal shape, provided, at its middle transversal or cross-section, with a throughgoing hole 2, which is open on the two main walls of this shaped body.

Within this hole 2 there is engaged a cylindrical bush 3, which is restrained in said hole by means of a pair of threaded stems 5 longitudinally formed in the body 1.

Said threaded stems are provided with and end piece 6 provided for engaging in a groove 7 which is formed along a perimetrical portion of said bush, and extend parallel to the longitudinal axis of said bush.

This bush, moreover, is provided with a throughgoing hole 8 the axis of which forms, with the transversal axis of said bush, a suitable angle which depends on the inclination of the diaphysis-femur axis of the patient with respect to a vertical axis.

The first shaped body 1 is moreover provided with two cross throughgoing holes 9 and, at the top face thereof 10, with two threaded counterbores indicated at 11.

In this connection, it should be apparent that, in said first shaped body there are furthermore formed, on the sides of the central throughgoing hole 2, at the top thereof, two coplanar slots 12 which are parallel to the top face of said first body, whereas, at the bottom of said first body, there are provided at least three pairs of slots 13, also coplanar but slightly downwardly with respect to the rear face of said first body provided for contacting the head of the femur.

In particular, to the mentioned top face of the body 1 there is coupled, through the interposition of two spacer cylindrical members 14, a small plate 15 provided with a lug 16 the end 17 of which is right angle bent.

Preferably, this small plate is coupled to the body 1 by means of two threaded holes 18 formed therethrough and by using, as the spacer cylindrical members, two tubular members provided with a top ridge 19, also threaded, provided for engaging in said holes.

Then, the thus formed assembly will be affixed on the top face of the body 1 through screws 20 engaging in the holes 11 formed on said face.

Thus, by suitably changing the height of the spacer cylindrical members 14,it will be possible to adjust the spacing between the small plate 18 and body 11,depending on the requirements.

The device further comprises a second shaped body 21,also having a substantially parallelepipedal shape,provided with a cantilever rectangular plate 22.

This plate 22 is provided with a central hole 23 and two side holes 24 arranged at and sized based on the throughgoing hole 2 and counterbore 11 formed on the top face of said body 1.

Through said second shaped body 21 there are moreover formed at least two cross throughgoing holes 25,as well as a pair of slots 26 which are mutually coplanar and perpendicular to the mentioned plate 22,and a pair of slanted slots 27.

For properly using the subject device, it is necessary to form,at the start of the operation, a hole 29 at the center of the infracondyl groove,by using a suitable pointed tool 28.

Then,by means of an endomidullar femoral nail 30,inserted into the hole of the bush 3 end into said hole 29,the body 1 is displaced so sa to contact the head of the femur.

Successively,the plate 15 is affixed on the top face of said body and the body,by sliding with respect to the bush (which is coupled to the mentioned endomidullar nail ),fits to the biassing force exerted by the end portion of the lug 16 on the neck of the femur.

After this operation,the body 1 is further restrained on the head of the femur,by means of a twice bent small plate 31,engaged in one of the slots 13 said body being locked by means of a pair of pins 32 which,through the holes 9,are driven in said head of the femur.

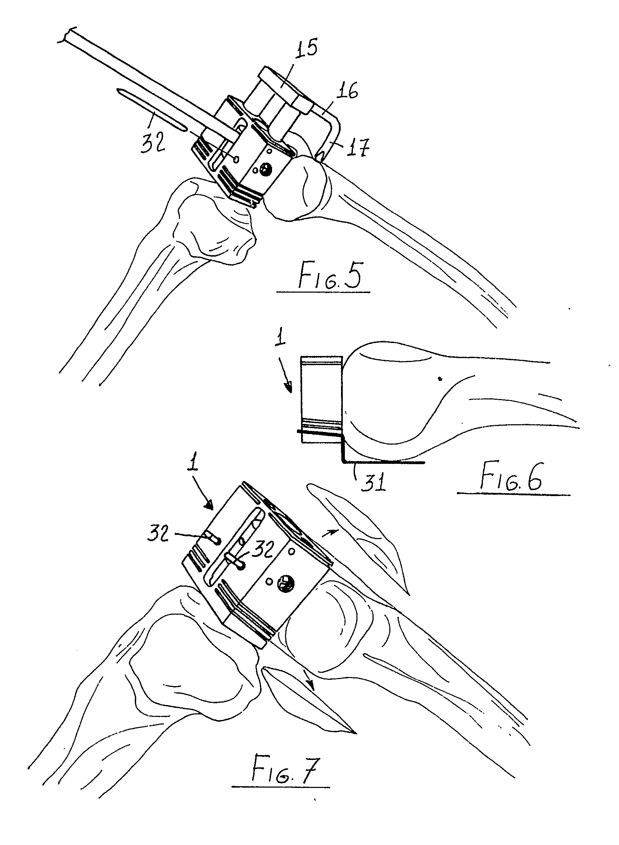

Then,the plate 15,with the related spacer members,and the small plate 31 are removed and the front and rear portions of the femur are resected as is shown in figure 7.

After this operation,the body 21 is affixed on the top face of the first shaped body 1 by means of screws 33 and said body 21 is locked on the front portion of the head of the femur,by means of pins 34 engaging the holes 25.

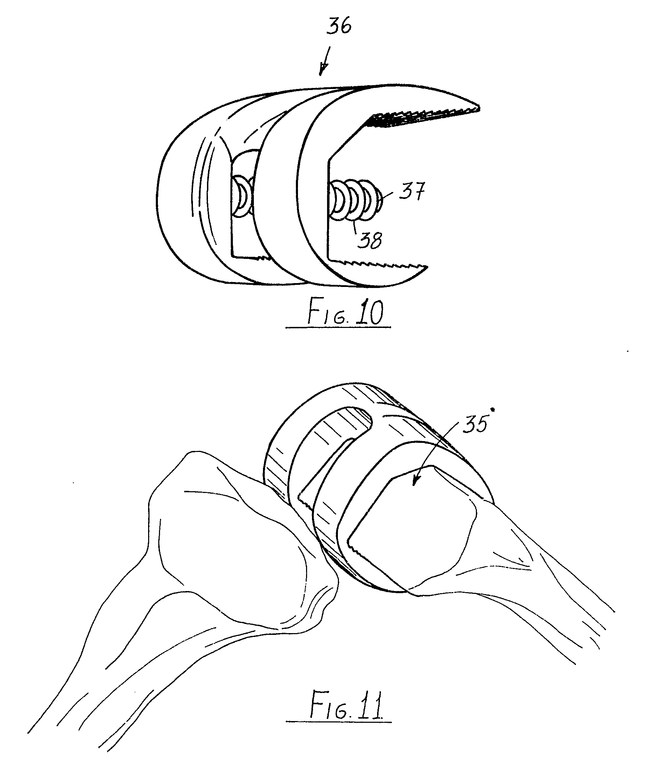

Then,the body 1 is removed and,by using the guiding slots 26 and 27,there are carried out the operations of transversely resecting the femoral condyles (as shown in figure 9) and the bevel 35.

Finally the prosthesis 36 is fitted on the head of the femur and locked thereon by forcing the pins 37,provided with annular ridges 38,into the holes 39 left by the pins 32.

From the above disclosure it should be apparent that the device according to the invention fully achieves the intended task and objects.

While the invention has been disclosed and illustrated with reference to a preferred embodiment thereof,it should be apparent that the disclosed embodiment is susceptible to several modifications and variations ,all of which will come within the spirit and scope of the appended claims.

## Claims

1- A femoral resection device characterized in that said device essentially comprises a first shaped body,having a substantially parallelepipedal shape, adapted to be affixed on the distal part of a femur, for the resection of the front and rear portions thereof,and a second shaped body,cooperating with the first shaped body and coupled thereto for transversely resecting said distal part of said femur.

2- A femoral resection device according to claim 1, characterized in that said first shaped body is provided,at its middle cross section,with a throughgoing hole,open on the two main walls of said first body and in which there is engaged a cylindrical bush restrained therein by a pair of threaded stems engaging in corresponding threaded holes longitudinally formed of said first shaped body.

3- A femoral resection device according to the preceding claims,characterized in that said threaded stems are provided with an end portion provided for engaging in a slot or groove formed along a perimetrical portion of said bush and extending parallel to the longitudinal axis of said bush,said bush being moreover provided with a throughgoing hole the axis of which forms a given angle with the cross axis of said bush,said angle depending on the inclination of the diaphysis-femur axis with respect to a vertical axis.

4- A femoral resection device,according to one or more of the preceding claims,characterized in that said first shaped body is provided with two cross throughgoing holes and,at the top face thereof,with two threaded counterbores,in said first shaped body there being moreover formed,on the sides of said central throughgoing hole,and at the top,two coplanar slots which are parallel to the top face of said first body, and,at the bottom,at least three pairs of further slots which are also coplanar but slightly downwardly slanted with respect to the rear face of said first shaped body provided for contacting said femur.

5- A femoral resection device according to one or more of the preceding claims,characterized in that to said top face of said first body there is coupled, through the interposition of spacer cylindrical members, a small plate having a lug the end free portion of which is right angle bent,said small plate being coupled to said first shaped body by means of two threaded holes and using,as said cylindrical spacer members, two tubular members provided with a top threaded ridge provided for engaging in said holes.

6- A femoral resection device,according to one or more of the preceding claims,characterized in that said device further comprises a second shaped body,of substantially parallelepipedal shape,provided with a cantilever rectangular plate including a central hole and two side holes.

7- A femoral resection device, according to one or more of the preceding claims, characterized in that in said second shaped body there are formed at least two cross throughgoing holes as well as a pair of slots coplanar with one another and perpendicular to said plate, and at least a pair of slanted slots.

8- A device according to any of the preceding claims and substantially as disclosed and illustrated for the intended task and objects.

$F_{IG}.1$

20

16

18

17

15

19

14

11

10

2

3

12

7

11

8

9

12

32

13

9

4

9

13

32

FIG. 2

28

FIG. 3

30

1

FIG. 4

F16.5

F16.6

F16.7

EP 0 340 176 A2

Fig. 8

Fig. 9

36

37

38

$\underline{F_{IG}.10}$

35

$\underline{F_{IG}.11}$